(19) Europäisches Patentamt
European Patent Office
Office européen des brevets

(11) EP 0 335 423 B2

(12) NEW EUROPEAN PATENT SPECIFICATION

(45) Date of publication and mention of the opposition decision:
**05.03.2003 Bulletin 2003/10**

(45) Mention of the grant of the patent:
**09.08.1995 Bulletin 1995/32**

(21) Application number: **89105728.3**

(22) Date of filing: **31.03.1989**

(51) Int Cl.[7]: **C07K 1/107**, C07K 14/535, C07D 251/26

(54) **Modified human G-CSF**

Modifizierter humaner G-CSF

G-CSF humain modifié

(84) Designated Contracting States:
**AT BE CH DE ES FR GB GR IT LI LU NL SE**

(30) Priority: **31.03.1988 JP 8008888**

(43) Date of publication of application:
**04.10.1989 Bulletin 1989/40**

(73) Proprietor: **KYOWA HAKKO KOGYO CO., LTD.**
**Chiyoda-ku, Tokyo-to (JP)**

(72) Inventors:
- **Yamasaki, Motoo**
  **Machida-shi Tokyo (JP)**
- **Yokoo, Yoshiharu**
  **Sagamihara-shi Kanagawa (JP)**
- **Morimoto, Makoto**
  **Sunto-gun Shizuoka (JP)**
- **Okabe, Masami**
  **Sunto-gun Shizuoka (JP)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**EP-A- 0 236 987** **EP-A- 0 251 717**

Remarks:
The file contains technical information submitted after the application was filed and not included in this specification

EP 0 335 423 B2

**Description**

FIELD OF THE INVENTION

This invention relates to a chemically modified hG-CSF polypeptide which results from chemical modification of at least one amino group in a polypeptide molecule having human granulocyte colony stimulating factor (hereinafter referred to briefly as hG-CSF) activity and a method of producing such modified polypeptides.

BACKGROUND OF THE INVENTION

hG-CSF is a polypeptide which is essential to the proliferation and differentiation of hematopoietic stem cells for the formation of various blood cells and has activity to promote mainly the multiplication of granulocytes, and particularly, of neutrophils. While neutrophils play a vital role in the protection of a living body against infection, they have a short lifetime and must be replenished at all times by the constant multiplication and differentiation of precursor cells. Therapies commonly practiced in recent years for proliferative tumors inhibit the proliferation of precursor cells of neutrophils as well, with the result that they suppress the anti-infective competence of tumor-bearing patients, a serious side effect. hG-CSF promotes the multiplication of neutrophils and, as such, is expected to alleviate this side effect and, at the same time, exert prophylactic and therapeutic effects against infections. Furthermore, hG-CSF has the ability to induce differentiation of cultured leukemia cells in vitro and, hence, has the potential for use as a drug for the treatment of leukemia. The chemically modified hG-CSF polypeptide of this invention has hG-CSF activity surpassing that of the known hG-CSF and is expected to be of value as a drug.

As a result of recombinant DNA technology, which has been developing rapidly in recent years, genes for proteinic factors involved in the proliferation and differentiation of blood cells have been isolated one after another. These factors are now produced by various genetic engineering techniques utilizing microbial or animal cells.

Regarding hG-CSF, Nagata et al. isolated cDNA from human squamous cell line CHU-II, determined its DNA sequence and reported on its expression in COS cells [Nagata et al.: Nature 319, 415 (1986)]. Moreover, Souza et al. isolated cDNA from human cystic cancer cell line 5637, determined its DNA sequence, and reported on its expression in Escherichia coli [Souza et al.: Science 232, 61 (1986)].

It has been reported that in administering the hG-CSF thus obtained to the body, a sustained effect can only be assured by repeated administration of hG-CSF and that discontinuation results in a rapid disappearance of the desired effect [Journal of Experimental Medicine 165, 941-948 (1987)]. This is probably due to the short life of hG-CSF in the blood.

In regard to enzymes such as asparaginase [Inada, Y. et. al.: Trends in Biotechnology 4, 68-73 (1986)], arginase [Savoca, K.V. et al.: Biochemica et Biophysica Acta 578, 47-53 (1979)], batroxobin [Nishimura, H. et al.: Life Science 33, 1467-1473 (1983)], etc., it has been found that chemical modification with polyethylene glycol results in an increased residence time in blood and in attenuated antigenicity.

In EP-A-0 251 717 chemically modified islet-activating protein (IAP) is disclosed. The modified product is obtained by reacting IAP with a triazine compound of the formula

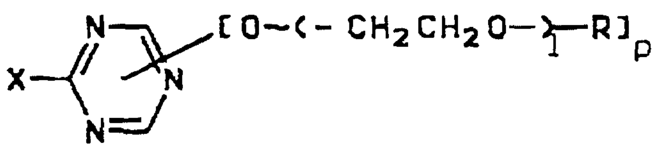

wherein R is a hydroxyl protective group, I is an integer of 7 to 700, p is 1 or 2, and X is halogen.

Protein is generally used as a drug in a lyophilized form. There is a problem that, during and/or after lyophilization, protein undergoes physioligocal or chemical changes, e.g., association, polymerization and oxidation due to external factors such as temperature, moisture, oxygen and ultraviolet light. Such changes often result in degradation of the activity of the protein. To overcome the above problem, stabilizers for protein during lyophilization have been investigated. For example, the stabilizers for hG-CSF are described in GB-A-2193631 and JP-A-63-146829 (the term "JP-A" as used herein means "an unexamined published Japanese patent application"). However, it has been required that the protein is further stabilized during and/or lyophilization for the clinical use.

In using hG-CSF as a drug, it is desirable that hG-CSF be stable and remain in the blood long after administration and its antigenicity be attenuated as well as stable during and/or after lyophilization for the practical use. However, there has not been an hG-CSF having such properties and, for that matter, a method for producing it.

## SUMMARY OF THE INVENTION

An object of the present invention is to provide a polypeptide having hG-CSF activity, which shows excellent stability and a long life in the blood.

The inventor of this invention has found that when at least one amino group in a polypeptide having hG-CSF activity is chemically modified, the resulting polypeptide stays longer in blood and stable during and/or after lyophilization than the unmodified polypeptide.

## DETAILED DESCRIPTION OF THE INVENTION

This invention provides a modified polypeptide having hG-CSF activity which is available on substitution of at least one amino group of a polypeptide having hG-CSF activity with a group of the formula

$$R_1\{OCH_2CH_2\}_nX—R_2— \qquad (I)$$

wherein $R_1$ is an alkyl or alkanoyl group; n is an optionally variable positive integer; X is O, NH or S; $R_2$ is

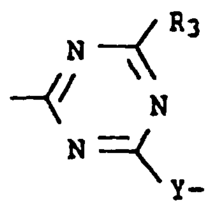

[where $R_3$ is OH, Cl, O—$(CH_2CH_2O)_n$—$R_1$ (where $R_1$and n are as defined above), Y may not be present or represents Z-$(CH_2)_p$CO, (where Z is O S or NH and p is an optionally variable positive integer)], or $(CO)_m$-$(CH_Z\ )_\ell$CO (where m is 0 or 1; $\ell$ is an optionally variable positive integer); provided that said modified polypeptide is not prepared from lysine depleted hG-CSF.

The starting polypeptide having hG-CSF activity may be any polypeptide having hG-CSF activity such as a polypeptide having the amino acid sequence shown in Table 1a, a polypeptide available upon replacement of at least one amino acid residue of the amino acid sequence shown in Table 1a with another kind of amino acid, e.g. the hG-CSF derivatives shown in Table 1b. or a polypeptide deficient in 1 to 11 amino acid residues at the N-terminus of the amino acid sequence shown in Table 1a. Aside from the above polypeptides. the hG-CSF derivatives described in EP-A-243153, EP-A-237545 and WO-A-8701132 can also be employed.

## Table 1a

X Thr Pro Leu Gly Pro Ala Ser Ser Leu Pro Gln Ser Phe Leu Leu Lys Cys Leu Glu
1 10

Gln Val Arg Lys Ile Gln Gly Asp Gly Ala Ala Leu Gln Glu Lys Leu Cys Ala Thr
20 30

Tyr Lys Leu Cys His Pro Glu Glu Leu Val Leu Leu Gly His Ser Leu Gly Ile Pro
40 50

Trp Ala Pro Leu Ser Ser Cys Pro Ser Gln Ala Leu Gln Leu Ala Gly Cys Leu Ser
60 70

Gln Leu His Ser Gly Leu Phe Leu Tyr Gln Gly Leu Leu Gln Ala Leu Glu Gly Ile
80 90

Ser Pro Glu Leu Gly Pro Thr Leu Asp Thr Leu Gln Leu Asp Val Ala Asp Phe Ala
100 110

Thr Thr Ile Trp Gln Gln Met Glu Glu Leu Gly Met Ala Pro Ala Leu Gln Pro Thr
120 130

Gln Gly Ala Met Pro Ala Phe Ala Ser Ala Phe Gln Arg Arg Ala Gly Gly Val Leu
140 150

Val Ala Ser His Leu Gln Ser Phe Leu Glu Val Ser Tyr Arg Val Leu Arg His Leu
160 170

Ala Gln Pro
174

(X = H or Met)

Table 1b

| Position of substitution (an amino acid of hG-CSF) | hG-CSF derivatives | | | | | | | | | | |
|---|---|---|---|---|---|---|---|---|---|---|---|
| | a) | b) | c) | d) | e) | f) | g) | h) | i) | j) | k) |
| Position-1 (Thr) | * | Val | Cys | Tyr | Arg | * | Asn | lie | Ser | * | Ala |

* No substitution

Table 1b (continued)

| Position of substitution (an amino acid of hG-CSF) | hG-CSF derivatives | | | | | | | | | | |
|---|---|---|---|---|---|---|---|---|---|---|---|
| | a) | b) | c) | d) | e) | f) | g) | h) | i) | j) | k) |
| Position-3 (Leu) | Glu | Ile | Ile | lie | Thr | Thr | Glu | Thr | Thr | * | Thr |
| Position-4 (Gly) | Lys | Arg | Arg | Arg | Arg | Arg | Arg | Arg | Arg | Arg | Tyr |
| Position-5 (Pro) | Ser | Ser | Ser | Ser | Ser | Ser | Ser | Ser | Ser | * | Arg |
| Position-17 (Cys) | Ser | Ser | Ser | Ser | Ser | Ser | Ser | Ser | Ser | Ser | Ser |

* No substitution

Referring to the chemically modifying group to be used in accordance with this invention, the alkyl and alkanoyl groups mentioned as protective groups for the terminal oxygen atom are $C_{1-18}$ alkyl groups (for example, methyl, ethyl, propyl, etc.) and $C_{1-18}$ alkanoyl groups (for example, formyl, acetyl, propionyl, etc.).
The positive integer n is not more than 500 and preferably 7 to 230.
The positive integer $\ell$ is not more than 100 and preferably 0 to 6. The positive integer p is from 1 to 18, preferably 1 to 6. The molecular weight of said chemically modifying group is not more than 30,000 and preferably in the range of 300 to 20,000.
The chemically modified hG-CSF of this invention is produced, for example, by condensation of hG-CSF with a halide of formula (II)

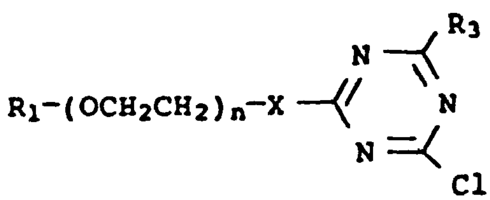

(II)

wherein $R_1$, n, X and $R_3$ are as defined hereinbefore or by condensation of hG-CSF with a carboxylic acid of formula (III)

$$R_1\{OCH_2CH_2)_n—X—(CO)_m(CH_2)_\ell—COOH \qquad (III)$$

wherein $R_1$, n, X, m and $\ell$ are as defined hereinbefore or a carboxyolic acid of formula (IV)

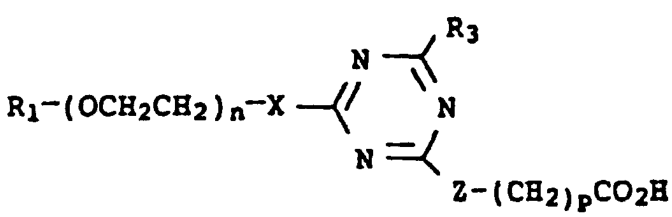

(IV)

wherein $R_1$, n, Z, X, $R_3$ and p are as defined hereinbefore; provided that condensation is not performed with lysine depleted hG-CSF.
The halide of formula (II) can be prepared by condensing

$$R_1\{OCH_2CH_2)_n—XH$$

(wherein $R_1$, n and X are as defined above) with cyanuric chloride [Matsushima, A. et al: Chemistry Letters, 773-776, 1980); Abuchowski, A. et al.: Journal of Biological Chemistry 252 (12) 3578-3581, 1977]. This halide is reactive and can therefore be directly reacted with a polypeptide having hG-CSF activity.
The carboxylic acid of formula (III) can be prepared by subjecting

$$R_1(OCH_2CH_2)_n\text{—}XH$$

wherein $R_1$, n and X are as defined hereinbefore, to dehydrative condensation with a carboxyl group of an alkanedicarboxylic acid or reaction with a halogenated monocarboxylic acid so as to introduce a carboxylic group or to an oxidation reaction of its terminal hydroxyl group to convert the latter to a carboxyl group. This carboxylic acid is not reactive and must, therefore, be activated before use. This activation of the carboxylic acid can for example be accomplished by converting it to an active ester with, for example, N-hydroxysuccinimide, N-hydroxyphthalimide, I-hydroxybenzotriazole, p-nitrophenol or the like, a mixed acid anhydride with isobutyl chloroformate, ethyl chloroformate or the like, or to an acid halide using a halogenating agent such as thionyl chloride. [All of the above methods are described, for example, in Peptide Gosei (Peptide Synthesis) (Nobuo Izumiya et al., Maruzen)].

The carboxylic acid of formula (IV) can be prepared by condensing the halide of formula (II) with $HZ\text{-}(CH_2)_pCO_2H$ (where Z and p are as defined above). This carboxylic acid of formula (IV) should be activated before use as well as that of formula (III).

The chemically modified hG-CSF of this invention is preferably produced by condensing hG-CSF with the carboxylic acid represented by formula (V)

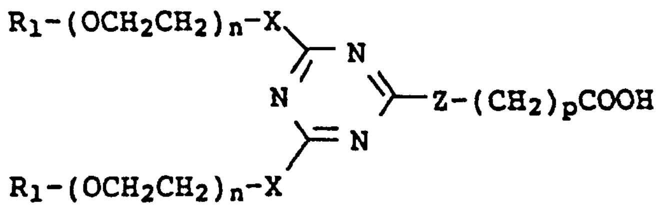

(V)

wherein $R_1$, n and X are as defined above, Z is O, S or NH and p is an optionally variable positive integer.

To this polypeptide having hG-CSF activity is added the above-mentioned halide or active carboxylic acid compound in a proportion (mole ratio) of 2 to 100 times the amount of amino groups present in the polypeptide molecule and the mixture is allowed to react at a temperature of 4 to 37°C, preferably 4 to 10°C, and pH 7 to 10 for 1 hour to 2 days, preferably 1 to 24 hours, whereby the desired chemically modified hG-CSF is produced.

The reaction products of hG-CSF or a derivative thereof with the halide of formula (II) and the carboxylic acids of formulae (III) and (IV) are hereinafter referred to as chemically modified hG-CSF (II), (III) and (IV), respectively.

The degree of chemical modification can be ascertained by quantitating the amount of decrease in free amino groups with trinitrobenzenesulfonic acid or monitoring a change in mobility of chemically modified hG-CSF by sodium dodecylsulfate (SDS)-polyacrylamide gel electrophoresis.

The chemically modified hG-CSF or a derivative thereof is used as a drug, i.e., an injectable solution, which is prepared by dissolving in water or an appropriate buffer and subjecting to filter-sterilization. When the modified hG-CSF of the present invention is lyophilized, the lyophilized product is also dissolved in water or an appropriate buffer and filter-sterilized to prepare an injectable solution.

The conditions at lyophilization are not particularly restricted. The lyophilization is generally carried out by freezing at -50°C or less for 1 to 5 hours, drying at -20°C to 0°C at a vacuum degree of 50 to 150 mTorr for 24 to 48 hours, and further drying at 10 to 30°C at a vacuum degree of 50 to 100 mTorr for 16 to 24 hours.

The preparation of chemically modified hG-CSF or a derivative thereof may contain additives such as pharmaceutically acceptable carries, vehicles, stabilizers or adsorption-preventing agents. The modified hG-CSF of the invention is administered to an adult in an amount of generally from 0.1 to 500 µg, preferably from 0.5 to 200 µg, 1 to 7 times a week. The dosage varies depending on the kind of disease and symptom of the patient.

According to the modified hG-CSF of the invention, 1 to 3 molecules of a polyethylene glycol (PEG) derivative are bound to each molecule (hereinafter referred to as mono-, di- and tri-type hG-CSF, respectively). The above-descirbed modified hG-CSF preparation may be a mixture of the mono-, di- and tri-type hG-CSF or these types of modified hG-CSF may be used as separated each other.

The determination of protein quantity in this invention is carried out by the following test methods.

Test method 1

The method of Lowry (Lowry, O. H. et al.: Journal of Biological Chemistry 193, 265, 1951).

Test method 2

The method of Laemmli (Laemmli, U.K.: Nature 227, 680, 1970) in which SDS-polyacrylamide gel electrophoresis is followed by determination with a chromatoscanner (CS-930, Shimadzu).

The determination of G-CSF activity in this invention was carried out in the following manner. Myelocytes were aseptically taken from the femoral bone of male C3H/He mice aged 8 to 12 weeks (purchased from Shizuoka Laboratory Animal Center) and suspended in α-Minimum Essential Medium (Flow Laboratories, hereinafter referred to briefly as α-MEM) containing 10% of fetal bovine serum (FBS). This cell suspension (about 5 x $10^7$ cells), 1.5 mℓ, was applied to a nylon wool (Nylon Fiber 146-04231, Wako Pure Chemical Industries, Ltd.) column (0.3 g) and incubated in a 5% $CO_2$ incubator at 37°C for 90 minutes. Then, α-MEM pre-warmed to 37°C was passed through the column, whereby myelocytes not adsorbed on nylon wool were obtained in the effluent. This cell fraction was washed with α-MEM once and adjusted to a specified concentration.

Then, in accordance with the method of Okabe et al. (Okabe, T. et al.: Cancer Research 44, 4503-4506, 1986), the assay of bone marrow hematopoietic cell colony-forming activity was performed. Thus, to a mixture of 0.2 mℓ of α-MEM, 0.4 mℓ of FBS and 0.2 mℓ of doubling sample dilutions was added 0.2 mℓ of the myelocytes (2 x $10^6$ cells/mℓ) prepared by the above procedure. The resulting mixture was further mixed with an equal volume (1.0 mℓ) of 0.6% agar (Difco, Agar purified No. 0560-01) solution maintained at 42°C and a 0.5 mℓ portion of the resulting mixture was distributed into a 24-well Multidish (Nunc, No. 143982) (5 x $10^4$ cells/well, n=3). The dish was maintained in a 5% $CO_2$ incubator at 37°C for 7 days and the number of colonies consisting of 40 or more cells was counted under the microscope (Olympus, X40). After this colony counting, each colony was carefully taken out on a glass slide, fixed with acetone-formalin for 30 seconds and subjected to esterase double-staining by the method of Kubota et al. (Kubota, K. et al.: Experimental hematology 8, 339-344, 1980) for identification.

The. potency of each sample was calculated from the counts for doubling dilutions in the colony forming test as follows. The activity value giving 1/2 the maximum colony forming value of G-CSF used as a standard was defined as 50 units and this value was multiplied by the dilution factor of each sample and, for conversion to activity per unit mℓ, by 20 to arrive at the potency (units) of the sample. The specific activity (units/mg) was expressed in potency per weight (mg) of protein.

The following examples, reference examples and experimental examples are further illustrative of this invention, but are not construed to limit the scope of the invention.

EXAMPLE 1

To 3 mℓ of 0.1 M borate buffer (pH 10) containing 186 μg/mℓ of hG-CSF with the amino acid sequence shown in Table 1a was added 56 mg of the chloro-compound prepared in Reference Example 1 and the reaction was carried out at 4°C for 24 hours with stirring.

The unreacted chloro-compound was removed by ultrafiltration (cutoff molecular weight 30,000) and, then, using YMC-Pack AM-312ODS (Kurita Industries, Ltd.), reversed phase HPLC on a linear gradient of 0 to 70% acetonitrile was carried out. The chemically modified hG-CSF polypeptide was eluted in the fraction of about 50% acetonitrile (yield 30 μg, percent yield 5%). It was confirmed by SDS-polyacrylamide gel electrophoresis that this chemically modified hG-CSF polypeptide had one chloro compound residue per molecule. The purity was in excess of 90%.

EXAMPLE 2

To 50 mℓ of 50 mM phosphate buffer (pH 7.2) containing 570 μg/mℓ of hG-CSF with the amino acid sequence shown in Table 1a was added 240 mg of the active ester prepared in Reference Example 2 and the reaction was carried out at 4°C for 6 hours with stirring.

After addition of 50 mℓ of 10 mM Tris-HCl buffer-0.7 M ammonium sulfate (pH 8.0), the reaction mixture was passed through a column (2.2 cm x 26 cm) of butyl-Toyopearl 650M (Tosoh) equilibrated with 10 mM Tris-HCl-0.35 M ammonium sulfate (pH 8.0) at a flow rate of 100 mℓ/hr. Then, the column was washed by passing 100 mℓ of 10 mM Tris-HCl-0.35 M ammonium sulfate (pH 8.0) at a flow rate of 100 mℓ/hr and, then, elution was carried out on a linear gradient with 200 mℓ of 10 mM Tris-HCl-0.35 M ammonium sulfate (pH 8.0) to 200 mℓ of 10 mM Tris-HCl buffer (pH 8.0) at a flow rate of 100 mℓ/hr. The object compound was eluted in fractions corresponding to 50 mM through 130 mM of ammonium sulfate. These fractions were collected (130 mℓ), subjected to ultrafiltration (cutoff molecular weight 10,000; membrane YM10 (Amicon), and concentrated to 7 mℓ. The concentrate obtained was passed through a column (2.8 cm x 70 cm) of Sephacryl S-200 (Pharmacia) equilibrated with 10 mM phosphate buffer-physiological saline (PBS) (pH 7.2) at a flow rate of 120 mℓ/hr, followed by passage of PBS at the same flow rate. The tri-type chemically modified hG-CSF polypeptide was eluted in fractions corresponding to 150 mℓ through 160 mℓ of PBS (yield 2 mg, percent yield 7%). The di- and mono-type modified hG-CSF polypeptides were subsequently eluted in fractions of 165 mℓ through

185 mℓ of PBS (yield 1.5 mg, percent yield 5%) and 190 mℓ through 210 mℓ of PBS (yield 4.5 mg, percent yield 16%). It was verified by SDS-polyacrylamide gel electrophoresis that, in the mono-type hG-CSF polypeptide, one molecule of the polyethylene glycol derivative carboxylic acid had been bound to each molecule of hG-CSF, two molecules in the di-type hG-CSF and three molecules in the tri-type hG-CSF. The purity of each polypeptide was not less than 90%.

## EXAMPLE 3

To 10 mℓ of 0.1 M borate buffer (pH 9) containing the hG-CSF derivative (570 μg/mℓ) obtained in Reference Example 3 was added 54 mg of the active ester obtained in Reference Example 2 and the reaction was conducted at 4 ° C for 10 hours with stirring.

The unreacted active ester and its decomposition product were removed with an ultrafiltration membrane YM30 (Amicon) and, then, the internal fluid was substituted with 10 mM Tris-HCl buffer (pH 8) using the same membrane. The residual fluid was passed through a column (1.7 cm x 4.4 cm) of DEAE-Toyopearl 650M (Tosoh) equilibrated with 10 mM Tris-HCl buffer (pH 8.0) at a flow rate of 10 mℓ/hr. Then, the column was washed by passing 20 mℓ of 10 mM Tris-HCl buffer (pH 8) at a flow rate of 5 mℓ/hr and, then, elution was carried out on a linear gradient with 50 mℓ of 10 mM Tris-HCl buffer (pH 8) to 10 mM Tris-HCl-0.4 M NaCl (pH 8) at a flow rate of 5 mℓ/hr. The chemically modified hG-CSF polypeptide was eluted in fractions corresponding to 100 through 120 mM of NaCl (yield 0.85 mg; percent yield 15%). It was verified by SDS-polyacrylamide gel electrophoresis that, in the resulting polypeptide, one molecule of the polyethylene glycol derivative carboxylic acid had been bound to one molecule of the hG-CSF derivative. The purity of this polypeptide was not less than 90%.

## EXAMPLE 4

To 50 mℓ of 50 mM phosphate buffer (pH 7.2) containing 570 μg/mℓ of the hG-CSF derivative obtained in Reference Example 3 was added 300 mg of the active ester prepared in Reference Example 2 and the reaction was carried out at 4°C for 6 hours with stirring.

After addition of 50 mℓ of 10 mM Tris-HCl buffer-0.7 M ammonium sulfate (pH 8.0), the reaction mixture was passed through a column (2.2 cm x 26 cm) of butyl-Toyopearl 650M (Tosoh) equilibrated with 10 mM Tris-HCl-0.35 M ammonium sulfate (pH 8.0) at a flow rate of 100 mℓ/hr. Then, the column was washed by passing 100 mℓ of 10 mM Tris-HCl-0.35 M ammonium sulfate (pH 8.0) at a flow rate of 100 mℓ/hr and, then, elution was carried out on a linear gradient of 10 mM Tris-HCl buffer (pH 8.0) and 400 mℓ of 0.35 M to 0 M of ammonium sulfate at a flow rate of 100 mℓ/hr. The object compound was eluted in fractions corresponding to 50 mM through 150 mM of ammonium sulfate. These fractions were collected (150 mℓ), subjected to ultrafiltration (cutoff molecular weight 10,000; membrane YM10 (Amicon), and concentrated to 10 mℓ. The concentrate obtained was passed through a column (2.8 cm x 70 cm) of Sephacryl S-200 (Pharmacia) equilibrated with PBS at a flow rate of 120 mℓ/hr, followed by passage of PBS at the same flow rate. The tri-type chemically modified hG-CSF polypeptide was eluted in fractions corresponding to 150 mℓ through 160 mℓ of PBS (yield 1.5 mg, percent yield 5%). The di- and mono-type modified hG-CSF polypeptides were subsequently eluted in fractions of 165 mℓ through 185 mℓ (yield 3 mg, percent yield 11%) and 190 mℓ through 210 mℓ (yield 4 mg, percent yield 14%). It was verified by SDS-polyacrylamide gel electrophoresis that, ir the mono-type polypeptide, one molecule of the polyethylene glycol derivative carboxylic acid had been bound to each molecule of hG-CSF, two molecules in the di-type polypeptide and three molecules in the tri-type polypeptide. The purity of each polypeptide was not less than 90%.

## EXAMPLE 5

To 100 mℓ of 50 mM phosphate buffer (pH 7.2) containing 300 μg/mℓ of the hG-CSF derivative obtained in Reference Example 3 was added 800 mg of the active ester prepared in Reference Example 4 and the reaction was carried out at 4 ° C for 24 hours with stirring.

After addition of 100 mℓ of 10 mM Tris-HCl buffer-0.7 M ammonium sulfate (pH 8.0), the reaction mixture was passed through a column (2.2 cm x 26 cm) of butyl-Toyopearl 650M (Tosoh) equilibrated with 10 mM Tris-HCl-0.35 M ammonium sulfate (pH 8.0) at a flow rate of 100 mℓ/hr. Then, the column was washed by passing 100 mℓ of 10 mM Tris-HCl-0.35 M ammonium sulfate (pH 8.0) at a flow rate of 100 mℓ/hr and, then, elution was carried out on a linear gradient of 10 mM Tris-HCl buffer (pH 8.0) and 400 mℓ of 0.35 M to 0 M of ammonium sulfate at a flow rate of 100 mℓ/hr. The object compound was eluted in fractions corresponding to 0 mM through 250 mM of ammonium sulfate. These fractions were collected (250 mℓ), subjected to ultrafiltration (cutoff molecular weight 10,000; membrane YM10 (Amicon), and concentrated to 10 mℓ. The concentrate obtained was passed through a column (5.6 cm x 40 cm) of Sephacryl S-200 (Pharmacia) equilibrated with PBS at a flow rate of 160 mℓ/hr, followed by passage of PBS at the same flow rate. The tri-type chemically modified hG-CSF polypeptide was eluted in fractions corresponding to 360 mℓ through

400 mℓ of PBS (yield 2.1 mg, percent yield 7%). The di- and mono-type modified hG-CSF polypeptides were subsequently eluted in fractions of 420 mℓ through 450 mℓ (yield 1.5 mg, percent yield 5%) and 500 mℓ through 530 mℓ (yield 1.5 mg, percent yield 5%). It was verified by SDS-polyacrylamide gel electrophoresis that, in the mono-type polypeptide, one molecule of the polyethylene glycol derivative carboxylic acid had been bound to each molecule of hG-CSF, two molecules in the di-type polypeptide and three molecules in the tri-type polypeptide. The purity of each polypeptide was not less than 90%.

EXAMPLE 6

Preparation of lyophilization product of chemically modified hG-CSF and storage stability thereof

In the same manner as in Example 2, the hG-CSF was reacted with the active ester prepared in Reference Example 2. The unreacted active ester and its decomposition product were removed with an ultrafiltration membrane YM30 (Amicon) and, then, the internal fluid was substituted with 50 mM phosphate buffer containing 1 M sodium chloride (pH 7.2) using the same membrane. The resulting solution containing 200 μg/mℓ of the desired modified hG-CSF derivative was subjected to lyophilization.

The lyophilization was carried out by inoculating the hG-CSF solution into glass vials, freezing the vials at -50°C or less for 2 hours, drying at -20°C at a vacuum degree of 100 mTorr for 24 hours and further drying at 20 ° C at a vacuum degree of 80 mTorr for 24 hours. As a control, a mixed solution of the hG-CSF and polyethylene glycol was lyophilized in the same manner as above. Each lyophilization product was allowed to stand at 65 ° C and sampled at timed intervals. The sampled lyophilization product was dissolved in 50 mM phosphate buffer

(pH 7.2) to determine the residual G-CSF activity in accordance with the above-described method. The results are shown in Table 2.

The residual activity means relative activity to the activity before lyophilization and defined as the following equation.

$$\text{Residual activity (\%)} = \frac{\text{Activity after storage at timed intervals}}{\text{Activity before lyophilization}} \times 100$$

Table 2

| Storage stability of lyophilized chemically modified hG-CSF (65 ° C) | | | | |
|---|---|---|---|---|
| Sample | Residual activity (%) at timed intervals | | | |
| | 6 hrs. | 1 day | 2 days | 7 days |
| hG-CSF | 68 | 26 | 2 | 2 |
| hG-CSF with PEG 1) | 53 | 40 | 6 | <1 |
| hG-CSF with PEG 2) | 48 | 33 | 21 | 13 |
| Chemically modified hG-CSF | 102 | 57 | 35 | 25 |

Notes
1) 2.5 parts by weight of PEG per part by weight of hG-CSF
2) 5 parts by weight of PEG per part by weight of hG-CSF

EXAMPLE 7

Preparation of lyophilization product of chemically modified hG-CSF and storage stability thereof

In the same manner as in Example 4, the hG-CSF derivative was reacted with the active ester prepared in Reference Example 2, and chemically modified hG-CSF derivative solution was obtained in the same manner as in Example 6. The lyophilization was carried out as described in Example 6 and each lyophilization product was allowed to stand at 37 ° C for 7 days. The results are shown in Table 3.

Table 3

| Storage stability of chemically modified hG-CSF (37°C, 7 days) | |
|---|---|
| Sample | Residual activity (%) |
| hG-CSF derivative | 85 |
| hG-CSF derivative with PEG 1) | 94 |
| hG-CSF derivative with PEG 2) | 88 |
| Chemically modified hG-CSF derivative | 100 |

Notes
1) 2.5 parts by weight of PEG per part by weight of hG-CSF
2) 5 parts by weight of PEG per part by weight of hG-CSF

REFERENCE EXAMPLE 1

Production of 2,4-bis(O-methoxypolyethylene glycol)-6-chloro-s-triazine

In 100 mℓ of dry toluene containing 10 g of anhydrous sodium carbonate was dissolved 20 g of monomethoxypolyethylene glycol having an average molecular, weight of 4000 (Nippon Oil and Fats) and the solution was heated at 110°C for 30 minutes. Then, 500 mg of cyanuric chloride was added and the mixture was heated at 110°C for 24 hours. The reaction residue was filtered off, followed by addition of 300 mℓ of petroleum ether to cause precipitation. The precipitate was washed with several portions of petroleum ether to recover 10 g of 2,4 bis(O-methoxypolyethylene glycol)-6-chloro-s-triazine (yield 50%).

REFERENCE EXAMPLE 2

Synthesis of monomethoxypolyethylene glycol succinyl-N-hydroxysuccinimide ester

To 50 mℓ of dry toluene were added 20 g of thoroughly dehydrated monomethoxypolyethylene glycol having an average molecular weight of 5000 (Union Carbide) and 2 g of succinic anhydride and the mixture was refluxed at 150°C for 5 hours. The toluene was distilled off under reduced pressure and the residue was thoroughly solubilized by addition of 30 mℓ of methylene chloride. To this was added 400 mℓ of dry ethyl ether to cause precipitation. The precipitate was recrystallized from methylene chloride-ethyl ether (volume ratio = 1:3) to recover 10 g (yield about 50%) of succinylated monomethoxypolyethylene glycol. This succinylated product (3.3 g) and 100 mg of N-hydroxysuccinimide were solubilized in 5 mℓ of dry methylene chloride, followed by addition of 200 mg of dicyclohexylcarbodiimide (DCC) with ice-cooling. The mixture was then stirred at room temperature for 20 hours. The byproduct dicyclohexylurea (DCU) was filtered off and ethyl ether was added to the filtrate to cause precipitation. The resulting precipitate was recrystallized from methylene chloride ethyl ether (volume ratio = 1:3) to recover 2.5 g (yield 72%) of monomethoxypolyethylene glycol succinyl-N-hydroxysuccinimide ester.

REFERENCE EXAMPLE 3

A hG-CSF derivative corresponding to the amino acid sequence shown in Table 1a but containing alanine in lieu of the threonine in position-1, threonine in lieu of the leucine in position-3, tyrosine in lieu of the glycine in position-4, arginine in lieu of the proline in position-5 and serine in lieu of the cysteine in position-17 was prepared by the following procedure.

Escherichia coli W3110 str A (Escherichia coli ECfBD28, FERM BP-1479) carrying a plasmid pCfBD28 containing a DNA coding for the above-mentioned hG-CSF derivative was cultured in LG Medium (prepared by dissolving 10 g of Bactotryptone, 5 g of yeast extract, 5 g of NaCl and 1 ℓ of glucose in 1 ℓ of water and adjusting the solution to pH 7.0 with NaOH) at 37°C for 18 hours. A 5-mℓ portion of this culture was inoculated into 100 mℓ of MCG Medium (0.6% of $Na_2HPO_4$, 0.3% of $KH_2PO_4$, 0.5% of NaCl, 0.5% of casamino acids, 1 mM of $MgSO_4$ and 4 µg/mℓ of vitamin $B_1$; pH 7.2) containing 25 µg/mℓ of tryptophan and 50 µg/mℓ of ampicillin and incubated at 30°C for 4 to 8 hours. Thereafter, 10 µg/mℓ of 3,6-indoleacrylic acid (hereinafter referred to briefly as IAA), a tryptophan inducer, was added and the incubation was continued for an additional period of 2 to 12 hours. The resulting culture was centrifuged at 8,000 rpm for 10 minutes to harvest the cells which were then washed with 30 mM NaCl-30 mM Tris-HCl buffer (pH 7.5). The washed cells were suspended in 30 mℓ of the same buffer solution as above and subjected to sonic disruption

(Branson Sonic Power Company's Sonifier, Cell Disruptor 200, output control 2) (10 minutes). The disrupted cell suspension was centrifuged at 9,000 rpm for 30 minutes to collect the cellular residue. From this cellular residue, the hG-CSF derivative was extracted, purified, solubilized and reconstituted by the method of Marston et al. [F.A.O. Marston et al.: Bio/Technology 2, 800 (1984)].

REFERENCE EXAMPLE 4

Production of N-hydroxysuccinimide ester (IVb) of 2,4-bis (o-methoxypolyethylene glycol)-6-(3-carboxypropylamino)-s-triazine (IVa)

The chloride-compound obtained in Reference Example 1 (500 mg) was dissolved in 9 mℓ of anhydrous tetrahydrofuran. This solution was added to 1 mℓ of anhydrous dimethylamide containing 10 mg of γ-amino butyric acid and 28 μℓ of triethylamine and the resulting mixture was stirred at room temperature for 16 hours. After drying the mixture under reduced pressure, 30 mℓ of methylene chloride and 15 mℓ of 10 mM phophate buffer (pH 10) were added thereto for partition.

The upper layer was adjusted to pH 1 with 2N HCl and 30 mℓ of methylene chloride was added thereto for the second partition. The lower layer was fractionated, dryed with anhydrous sodium sulfate and subjected to filtration. The filtrate was concentrated under reduced pressure to obtain 150 mg of the carboxylic acid (IVa) (percent yield 30%). The thus-obtained carboxylic acid (IVa) (150 mg) and N-hydroxysuccinimide (3 mg) were solubilized in 1 mℓ of dry methylene chloride, followed by addition of 6 mg of DCC with ice-cooling. The mixture was then stirred at room temperature for 12 hours. The byproduct DCU was filtered off and ethyl ether was added to the filtrate to cause precipitation. The thus-formed precipitate was collected by filtration and dryed under reduced pressure to obtain 100 mg of the desired ester (IVb) (percent yield 67%).

TEST EXAMPLE 1

Specific activity and mouse leukemia cell NFS60 growth promoting activity of the chemically modified hG-CSF (III)

In the same manner as Example 3, the hG-CSF derivative was reacted with the active ester and the unreacted active ester and its decomposition product were removed using an ultrafiltration membrane. Then, using the same membrane as above, the internal fluid was substituted with PBS and the G-CSF activity and NFS60 cell growth promoting activity [Proceedings of the National Academy of Sciences of the USA 82, 6687 (1985)] of the chemically modified hG-CSF derivative in the residual fluid were assayed. The results are shown in Table 4.

Table 4

| Sample | Specific activity (unit/mg protein) | NFS60 growth promoting activity |
|---|---|---|
| hG-CSF derivative | 100 % | 100 % |
| Chemically modified hG-CSF derivative | 12.9 % | 6.9 % |

It is evident from the above results that the chemically modified hG-CSF derivative retained CSF activity against mouse bone marrow stem cells. It is also clear that the same derivative had a growth promoting effect on NFS60 cells which are known to show G-CSF-dependent growth.

TEST EXAMPLE 2

Leukocyte (granulocyte) increasing effect

The same chemically modified hG-CSF (III) as used in Test Example 1 was subcutaneously administered to C3H/He mice (male; n=3) either once or once a day for 6 consecutive days. The blood was sampled at timed intervals and the white blood cells (WBC) in peripheral blood were counted. The results are shown in Table 5 (single administration) and Table 6 (repeated administration).

Table 5

| The time course of WBC after single administration (s.c.) | | | | | | | | |
|---|---|---|---|---|---|---|---|---|
| Sample | Dosage a) (µg/ mouse) | WBC (% of normal control) Blood sampling interval (hr.) | | | | | | |
| | | 1 | 5 | 8 | 16 | 24 | 48 | 72 |
| hG-CSF derivative | 10 | 75.4 | 159.1 | 228.3 | 166.7 | 200.1 | 125.5 | 110.0 |
| Chemically modified hG-CSF derivative | 10 | 81.1 | 179.2 | 259.9 | 169.8 | 186.7 | 177.4** | 96.5 |

Notes
a) The same weight as hG-CSF protein was administered.
**) P<0.01 (Student's t-test)

Table 6

| The time course of WBC in 6-day repeated administration (s.c.) | | | | | | | |
|---|---|---|---|---|---|---|---|
| Sample | Dosage a) (µg/mouse /day) | WBC (% of normal control) Blood sampling interval (Day) | | | | | |
| | | 1 | 2 | 3 | 4 | 5 | 6 |
| hG-CSF derivative | 1 | 79.3 | 95.5 | 85.1 | 91.2 | 79.1 | 116.8 |
| Chemically modified hG-CSF derivative | 1 | 131.1** | 185.4*** | 148.7*** | 125.9* | 124.4* | 143.4 |
| hG-CSF derivative | 10 | 163.0 | 221.5 | 220.3 | 289.3 | 273.0 | 284.0 |
| Chemically modified hG-CSF derivative | 10 | 120.9 | 181.3 | 171.5 | 273.1 | 355.4 | 442.3 |

Notes
a) The same weight as hG-CSF protein was administered.
*) P<0.05,
**) P<0.01,
***) P<0.001 (Student's t-test)

In single administration, increase in WBC peaking at 8 hours after administration were observed but whereas the count declined thereafter to normal in 48 hours after administration in the case of the hG-CSF derivative, a significant increase in WBC was still observed even after 48 hours in the case of the chemically modified hG-CSF derivative.

In repeated administration, particularly in the low dose group, the chemically modified hG-CSF derivative showed a significant leukocyte increasing effect as compared with the hG-CSF derivative.

TEST EXAMPLE 3

Time course of plasma concentration

The chemically modified hG-CSF derivative as used in Test Example 1 was subcutaneously administered to C3H/He mice (male, n=3) either once or once a day for 6 consecutive days. The blood was sampled at timed intervals and the plasma concentration of G-CSF was determined. The results are set forth in Table 7 (single administration) and Table 8 (repeated administration). In some experiments, a single dose of the same chemically modified hG-CSF derivative was intravenously administered (Table 9).

Table 7

| Single administration (s.c.) | | | | | | | | |
|---|---|---|---|---|---|---|---|---|
| Sample | Dosage a) (μg/mouse/day) | Plasma concentration (Units/mℓ b) plasma, x $10^4$) Blood sampling interval | | | | | | |
| | | 15 min | 30 min | 1 hr | 5 hr | 7.5 hr | 15 hr | 24 hr |
| hG-CSF derivative | 10 | 248.3 | 772.7 | 2744.5 | 214.0 | 163.3 | 49.7 | 6.2 |
| Chemically modified hG-CSF derivative | 10 | 29.8 | 44.6 | 208.3 | 1709.0 | 1146.7 | 89.6 | 21.6 |

Notes
a) The same weight as G-CSF protein was administrered.
b) Calculated from NFS 60 cell growth promoting acitivity (Half max = 50 U).

Table 8

| Repeated administration (s.c.) | | | | | | | |
|---|---|---|---|---|---|---|---|
| Sample | Dosage a) (μg/mouse/day) | Plasma concentration b) (Units/mℓ c) plasma, x $10^{4)}$ Blood sampling interval (Day) | | | | | |
| | | 0 | 1 | 2 | 3 | 4 | 5 |
| hG-CSF derivative | 10 | NT.d)<br>4.8 | 1354.9<br>2.2 | 692.7<br>NT. | 915.3<br>-e) | 768.8<br>- | 756.4<br>- |
| Chemically modified hG-CSF derivative | 10 | NT.<br>14.2 | 92.2<br>11.3 | 376.9<br>NT. | 235.9<br>4.7 | 53.7<br>2.2 | 53.9<br>2.2 |

Notes
a) The same weight as G-CSF protein was administered.
b) Upper row: plasma concentration at 1 hr after administration Lower row: plasma concentration at 24 hr after administration
c) calculated from NFS 60 cell growth promoting activity (Half max = 50 U)
d) NT. (not tested)
e) - below detection limit

Table 9

| Single administration (i.v.) | | | | | | | |
|---|---|---|---|---|---|---|---|
| Sample | Dosage a) (μg/mouse/day) | Plasma concentration (Units/mℓ b) plasma, x $10^4$) Blood sampling interval | | | | | |
| | | 3 min | 10 min | 30 min | 1 hr | 2 hr | 5 hr |
| hG-CSF derivative | 10 | 1307 | 1356 | 901 | 631.3 | 563 | 355.8 |

Notes
a) The same weight as G-CSF protein was administered.
b) calculated from NFS 60 cell growth promoting activity (Half max = 50 U).

Table 9 (continued)

| Single administration (i.v.) | | | | | | | |
|---|---|---|---|---|---|---|---|
| Sample | Dosage a) (μg/ mouse/day) | Plasma concentration (Units/mℓ b) plasma, x $10^4$) Blood sampling interval | | | | | |
| | | 3 min | 10 min | 30 min | 1 hr | 2 hr | 5 hr |
| Chemically modified hG-CSF derivative | 10 | 6883 | 6181 | 4320 | 3332 | 1621 | 905.6 |

Notes
a) The same weight as G-CSF protein was administered.
b) calculated from NFS 60 cell growth promoting activity (Half max = 50 U).

In the case of single subcutaneous administration, whereas the plasma concentration of the hG-CSF deviative reached a peak at 1 hour and declined rapidly thereafter, that of the chemically modified hG-CSF derivative showed a gradual increase in 5 to 7 hours after administration and maintained a comparatively high level even after 24 hours (Table 7). On the other hand, in repeated subcutaneous administration, the hG-CSF derivative showed a higher plasma concentration at 1 hour after administration but a lower level at 24 hours and was no longer detected on day 3. In contrast, the chemically modified hG-CSF derivative was detectable even at 24 hours and its concentration was higher than that of the hG-CSF derivative.

In intravenous administration, the chemically modified hG-CSF administration, the chemically modified hG-CSF derivative gave significantly higher plasma concentrations as shown in Table 9.

TEST EXAMPLE 4

Specific activity and mouse leukemia cell NFS60 growth promoting activity of the chemically modified hG-CSF derivative (III)

(1) The chemically modified hG-CSG (III) obtained in Example 2 was assayed in the same manner as in Test Example 1. The results are shown in Table 10.

Table 10

| Sample | Specific activity (unit/mg protein) | NFS60 growth promoting activity |
|---|---|---|
| Unmodified hG-CSF | 100 % | 100 % |
| hG-CSF (III) mono-type | 58.0 % | 50.8 % |
| hG-CSF (III) di-type | 25.8 % | 35.0 % |
| hG-CSF (III) tri-type | 18.2 % | 21.0 % |

(2) In addition, the chemically modified hG-CSFs (III) and (IV) obtained in Examples 4 and respectively, were assayed as above. The results are shown in Table 11.

Table 11

| Sample | Specific activity (unit/mg protein) | NFS60 growth promoting activity |
|---|---|---|
| Unmodified hG-CSF | 100 % | 100 % |
| hG-CSF (III) mono-type | 60.0 % | 46.9 % |
| hG-CSF (III) di-type | 28.2 % | 24.6 % |
| hG-CSF (III) tri-type | 14.7 % | 19.0 % |
| hG-CSF (IV) mono-type | 68.4 % | 65.9 % |
| hG-CSF (IV) di-type | 22.2 % | 44.6 % |
| hG-CSF (IV) tri-type | 11.9% | 17.6 % |

TEST EXAMPLE 5

Leukocyte (granulocyte) increasing effect

(1) The chemically modified hG-CSF (III) obtained in Example 2 was subcutaneously administered to BALB/c mice (male, n=3; control group, n = 4) in an mount of 2.5 μg per animal. The blood was sampled at timed intervals and the WBC in peripheral blood were counted. The results are shown in Table 12.

Table 12

| The time course of WBC in single administration (s.c.) | | | | | |
|---|---|---|---|---|---|
| Sample | WBC (% of normal control) Blood sampling interval (hr.) | | | | |
| | 7 | 25 | 32 | 50 | 72 |
| Unmodified hG-CSF | 150 | 132 | 106 | 107 | 100 |
| hG-CSF (III) mono-type | 161 | 109 | 134 | 86 | 101 |
| hG-CSF (III) di-type | 174 | 166 | 176 | 113 | 91 |
| hG-CSF (III) tri-type | 161 | 130 | 152 | 133 | 82 |

(2) In the same manner as above, the chemically modified hG-CSFs (III) and (IV) obtained in Examples 4 and 5, respectively, were assayed. The results are shown in Table 13.

Table 13

| The time course of WBC in single administration (s.c.) | | | | | |
|---|---|---|---|---|---|
| Sample | WBC (% of normal control) Blood sampling interval (hr.) | | | | |
| | 7 | 25 | 32 | 50 | 72 |
| Unmodified hG-CSF | 143 | 131 | 140 | 104 | 118 |
| hG-CSF (III) mono-type | 161 | 152 | 143 | 108 | 137 |
| hG-CSF (III) di-type | 163 | 120 | 200 | 117 | 120 |
| hG-CSF (III) tri-type | 184 | 128 | 185 | 131 | 137 |
| hG-CSF (IV) mono-type | 153 | 183 | 233 | 124 | 104 |
| hG-CSF (IV) di-type | 120 | 156 | 212 | 169 | 110 |
| hG-CSF (IV) tri-type | 122 | 154 | 168 | 217 | 136 |

Thus, the chemically modified hG-CSF and chemically modified hG-CSF derivatives of this invention produce an enhanced peripheral leukocyte (granulocyte) increasing effect with improved stability and residence time in the blood and, as such, can be used advantageously in clinical medicines, e.g., a leukocyte growth promoting agent.

## Claims

**Claims for the following Contracting States : AT, BE, CH, DE, FR, GB, IT, LI, LU, NL, SE**

1. A modified polypeptide having human granulocyte colony stimulating factor (hG-CSF) activity comprising a polypeptide having hG-CSF activity with at least one amino group thereof substituted with a group of the formula

$$R_1\{OCH_2CH_2\}_nX\text{-}R_2\text{-} \qquad (I)$$

wherein $R_1$ is an alkyl or alkanoyl group; n is an optionally variable positive integer; X is O, NH or S; $R_2$ is

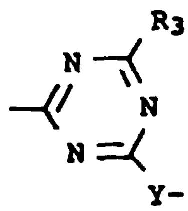

[where $R_3$ is OH, Cl, O-$(CH_2CH_2O)_n$-$R_1$, where $R_1$ and n are as defined above, Y may not be present or represents Z-$(CH_2)_p$CO, where Z is O, S or NH and p is an optionally variable positive integer], or (CO)-$_m$-$(CH_2)_1$CO, where m is 0 or 1; l is an optionally variable positive integer, provided that said modified polypeptide is not prepared from lysine depleted hG-CSF.

**2.** The modified polypeptide of claim 1, wherein $R_1$ is a $C_{1-18}$ alkyl or alkanoyl group.

**3.** The modified polypeptide of claim 1, wherein n is a positive integer of not more than 500.

**4.** The modified polypeptide of claim 1, wherein l is a positive integer of not more than 100.

**5.** The modified polypeptide of claim 1, wherein the group of formula (I) has a molecular weight of not more than 30,000.

**6.** A pharmaceutical composition comprising the modified polypeptide of anyone of claims 1 to 5, optionally in combination with a pharmaceutically acceptable carrier.

**7.** The pharmaceutical composition of claim 6 containing the modified polypeptide of anyone of claims 1 to 5 in lyophilized form.

**8.** A process for preparing a chemically modified hG-CSF according to anyone of claims 1 to 5, comprising condensation of hG-CSF with a halide of formula (II)

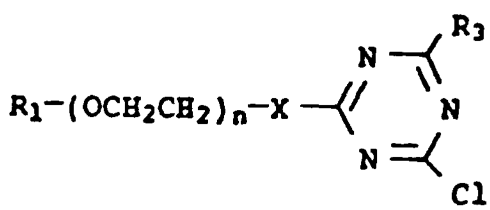

(II)

wherein $R_1$, n, X and $R_3$ are as defined hereinbefore;or condensation of hG-CSF with a carboxylic acid of formula (III)

$$R_1\{OCH_2CH_2)_n\text{-}X\text{-}(CO)_m(CH_2)_1\text{-}COOH \qquad (III)$$

wherein $R_1$, n, X, m and l are as defined hereinbefore;or with a carboxylic acid of formula (IV)

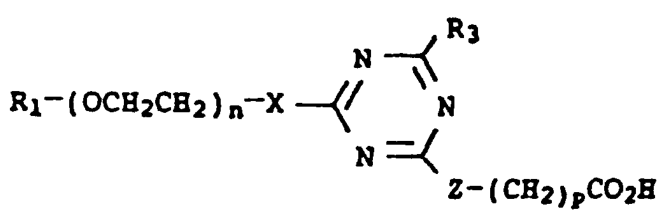

(IV)

wherein $R_1$, n, Z, X, $R_3$ and p are as defined hereinbefore; provided that condensation is not performed with lysine depleted hG-CSF.

**9.** The process according to claim 8, comprising condensation of hG-CSF with a carboxylic acid represented by formula (V)

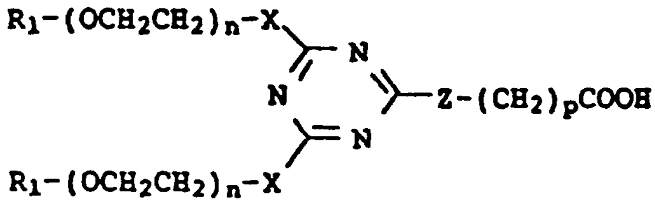

(V)

wherein $R_1$, n and X are as defined above, Z is O, S or NH and p is an optionally variable positive integer.

**10.** The process according to claim 8 or 9, **characterized in that** the halide or active carboxylic acid compound is added to the polypeptide in a proportion (mole ratio) of 2 to 100 times the amount of amino groups present in the polypeptide molecule and the mixture is allowed to react at a temperature of 4 to 37°C, preferably 4 to 10°C, and at pH 7 to 10 for 1 hour to 2 days, preferably 1 to 24 hours, whereby the desired chemically modified hG-CSF is produced.

**11.** The use of at least one modified polypeptide of claims 1 to 5 for preparing a pharmaceutical composition showing leukocyte growth promoting activity.

**Claims for the following Contracting State : ES**

**1.** A process for preparing a modified polypeptide having human granulocyte colony stimulating factor (hG-CSF) activity comprising a polypeptide having hG-CSF activity with at least one amino group thereof substituted with a group of the formula

$$R_1\{OCH_2CH_2\}_nX\text{-}R_2\text{-} \qquad (I)$$

wherein $R_1$ is an alkyl or alkanoyl group; n is an optionally variable positive integer; X is O, NH or S; $R_2$ is

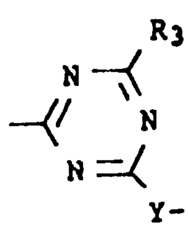

[where $R_3$ is OH, Cl, O-$(CH_2CH_2O)_n$-$R_1$, where $R_1$ and n are as defined above, Y may not be present or represents Z-$(CH_2)_p$CO, where Z is O, S or NH and p is an optionally variable positive integer], or $(CO)_{-m}$-$(CH_2)_1$CO, where m is 0 or 1; l is an optionally variable positive integer; which process comprises condensation of hG-CSF with a halide of formula (II)

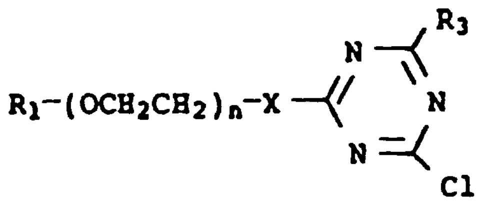

(II)

wherein $R_1$, n, X and $R_3$ are as defined hereinbefore; or condensation of hG-CSF with a carboxylic acid of formula (III)

$$R_1\{OCH_2CH_2)_n\text{-X-}(CO)_m(CH_2)_1\text{-COOH} \qquad \text{(III)}$$

wherein $R_1$, n, X, m and l are as defined hereinbefore; or with a carboxylic acid of formula (IV)

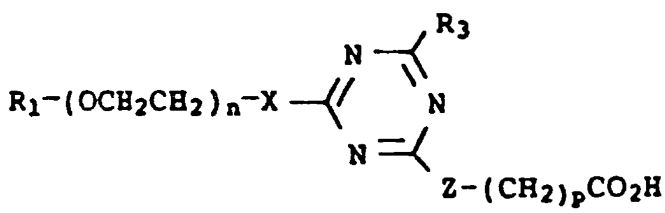

(IV)

wherein $R_1$, n, Z, X, $R_3$ and p are as defined hereinbefore; provided that condensation is not performed with lysine depleted hG-CSF.

**2.** The process according to claim 1, comprising condensation of hG-CSF with a carboxylic acid represented by formula (V)

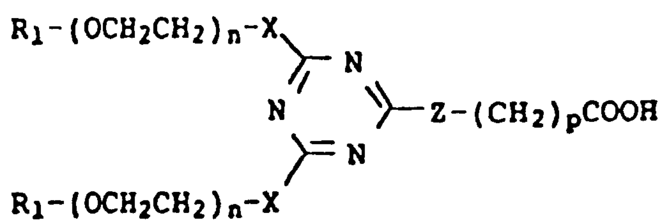

(V)

wherein $R_1$, n and X are as defined above, Z is O S or NH and p is an optionally variable positive integer.

**3.** The process according to claim 1 or 2, **characterized in that** the halide or active carboxylic acid compound is added to the polypeptide in a proportion (mole ratio) of 2 to 100 times the amount of amino groups present in the polypeptide molecule and the mixture is allowed to react at a temperature of 4 to 37°C, preferably 4 to 10°C and at pH 7 to 10 for 1 hour to 2 days, preferably 1 to 24 hours, whereby the desired chemically modified hG-CSF is produced.

**4.** The process of one of the claims 1 to 3, wherein $R_1$ is a $C_{1\text{-}18}$ alkyl or alkanoyl group.

**5.** The process of one of the claims 1 to 3, wherein n is a positive integer of not more than 500.

**6.** The process of one of the claims 1 to 3, wherein l is a positive integer of not more than 100.

**7.** The process of one of the claims 1 to 3, wherein the group of formula (I) has a molecular weight of not more than 30,000.

**8.** A process for preparing a pharmaceutical composition which process comprises providing a modified polypeptide as defined in anyone of claims 1 to 7, optionally in combination with a pharmaceutically acceptable carrier.

**9.** The process of claim 8 which process comprises providing the modified polypeptide as defined in anyone of claims 1 to 7 in lyophilized form.

**Claims for the following Contracting State : GR**

**1.** A modified polypeptide having human granulocyte colony stimulating factor (hG-CSF) activity comprising a polypeptide having hG-CSF activity with at least one amino group thereof substituted with a group of the formula

$$R_1\{OCH_2CH_2\}_nX\text{-}R_2\text{-} \qquad (I)$$

wherein $R_1$ is an alkyl or alkanoyl group; n is an optionally variable positive integer; X is O, NH or S; $R_2$ is

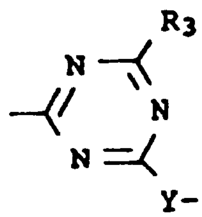

[where $R_3$ is OH, Cl, O-$(CH_2CH_2O)_n$-$R_1$, where $R_1$ and n are as defined above, Y may not be present or represents Z-$(CH_2)_p$CO, where Z is O, S or NH and p is an optionally variable positive integer], or $(CO)\text{-}_m\text{-}(CH_2)_1CO$, where m is 0 or 1; I is an optionally variable positive integer; provided that said modified polypeptide is not prepared from lysine depleted hG-CSF.

**2.** The modified polypeptide of claim 1, wherein $R_1$ is a $C_{1\text{-}18}$ alkyl or alkanoyl group.

**3.** The modified polypeptide of claim 1, wherein n is a positive integer of not more than 500.

**4.** The modified polypeptide of claim 1, wherein I is a positive integer of not more than 100.

**5.** The modified polypeptide of claim 1, wherein the group of formula (I) has a molecular weight of not more than 30,000.

**6.** A process for preparing a pharmaceutical composition which process comprises providing a modified polypeptide as defined in anyone of claims 1 to 5, optionally in combination with a pharmaceutically acceptable carrier.

**7.** The process of claim 6 which process comprises providing the modified polypeptide as defined in anyone of claims 1 to 5 in lyophilized form.

**8.** A process for preparing a chemically modified hG-CSF according to anyone of claims 1 to 5, comprising condensation of hG-CSF with a halide of formula (II)

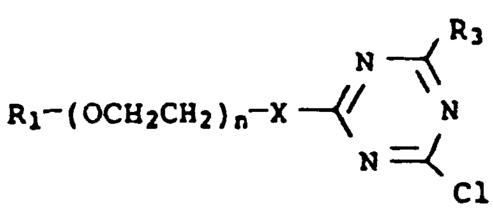

(II)

wherein $R_1$, n, X and $R_3$ are as defined hereinbefore; or condensation of hG-CSF with a carboxylic acid of formula (III)

$$R_1\{OCH_2CH_2)_n\text{-}X\text{-}(CO)_m(CH_2)_1\text{-}COOH \qquad \text{(III)}$$

wherein $R_1$, n, X, m and I are as defined hereinbefore; or with a carboxylic acid of formula (IV)

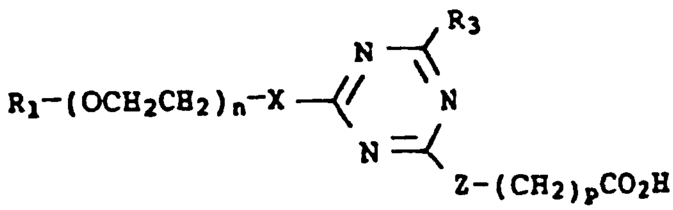

(IV)

wherein $R_1$, n, Z, X, $R_3$ and p are as defined hereinbefore; provided that condensation is not performed with lysine depleted hG-CSF.

**9.** The process according to claim 8, comprising condensation of hG-CSF with a carboxylic acid represented by formula (V)

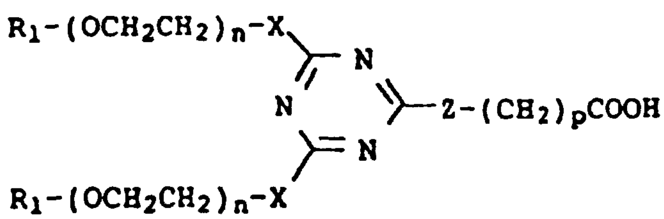

(V)

wherein $R_1$, n and X are as defined above, Z is O, S or NH and p is an optionally variable positive integer.

**10.** The process according to claim 8 or 9, **characterized in that** the halide or active carboxylic acid compound is added to the polypeptide in a proportion (mole ratio) of 2 to 100 times the amount of amino groups present in the polypeptide molecule and the mixture is allowed to react at a temperature of 4 to 37°C, preferably 4 to 10°C,and at pH 7 to 10 for 1 hour to 2 days, preferably 1 to 24 hours, whereby the desired chemically modified hG-CSF is produced.

**11.** The use of at least one modified polypeptide of claims 1 to 5 for preparing a pharmaceutical composition showing leukocyte growth promoting activity.

**Patentansprüche**

**Patentansprüche für folgende Vertragsstaaten : AT, BE, CH, DE, FR, GB, IT, LU, NL, SE**

**1.** Modifiziertes Polypeptid mit Aktivität von humanem Granulozyten-Kolonie-stimulierenden Faktor (hG-CSF), umfassend ein Polypeptid mit hG-CSF-Aktivität, wobei wenigstens eine Aminogruppe davon mit einer Gruppe der Formel

$$R_1\{OCH_2CH_2\}_nX\text{-}R_2\text{-} \qquad \text{(I)}$$

substituiert ist, worin

$R_1$ für eine Alkyl- oder Alkanoylgruppe steht;
n für einen gegebenenfalls variablen, positiven, ganzzahligen Wert steht;
X für O, NH oder S steht;
$R_2$ für

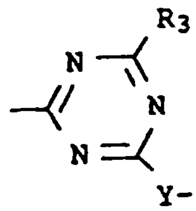

steht, [worin $R_3$ für OH, Cl, O-$(CH_2CH_2O)_n$-$R_1$ steht, worin $R_1$ und n die oben angegebenen Bedeutungen besitzen, Y fehlt oder für Z-$(CH_2)_p$CO steht, worin Z für O, S oder NH steht und p für einen gegebenenfalls variablen, positiven, ganzzahligen Wert steht]; oder für $(CO)_m$-$(CH_2)_1$CO steht, worin m für 0 oder 1 steht; und l für einen gegebenenfalls variablen, positiven, ganzzahligen Wert steht, mit der Maßgabe, dass das modifizierte Polypeptid nicht aus Lysin-abgereichertem hG-CSF hergestellt ist.

2. Modifiziertes Polypeptid nach Anspruch 1, worin $R_1$ für eine $C_{1-18}$-Alkyl- oder -Alkanoylgruppe steht.

3. Modifiziertes Polypeptid nach Anspruch 1, worin n für einen positiven, ganzzahligen Wert steht, der nicht größer als 500 ist.

4. Modifiziertes Polypeptid nach Anspruch 1, worin l für einen positiven, ganzzahligen Wert steht, der nicht größer als 100 ist.

5. Modifiziertes Polypeptid nach Anspruch 1, worin die Gruppe der Formel (I) ein Molekulargewicht von nicht mehr als 30000 besitzt.

6. Pharmazeutisches Mittel, umfassend ein modifiziertes Polypeptid gemäß einem der Ansprüche 1 bis 5, gegebenenfalls in Kombination mit einem pharmazeutisch akzeptablen Träger.

7. Pharmazeutisches Mittel nach Anspruch 6, enthaltend das modifizierte Polypeptid gemäß einem der Ansprüche 1 bis 5 in lyophilisierter Form.

8. Verfahren zur Herstellung eines chemisch modifizierten hG-CSF gemäß einem der Ansprüche 1 bis 5, umfassend die Kondensation von hG-CSF mit einem Halogenid der Formel (II)

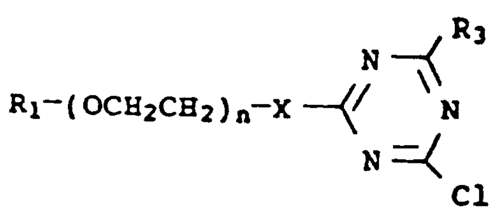

(II)

worin $R_1$, n, X und $R_3$ die oben angegebenen Bedeutungen besitzen; oder
die Kondensation von hG-CSF mit einer Carbonsäure der Formel (III)

$$R_1\{OCH_2CH_2)_n\text{-}X\text{-}(CO)_m(CH_2)_1\text{-}COOH \qquad \text{(III)}$$

worin $R_1$, n, X, m und l die oben angegebenen Bedeutungen besitzen; oder mit einer Carbonsäure der Formel (IV)

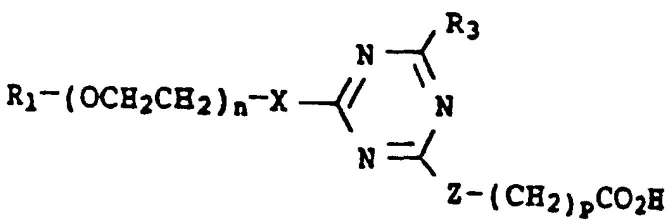

(IV)

worin $R_1$, n, Z, X, $R_3$ und p die oben angegebenen Bedeutungen besitzen, mit der Maβgabe, dass die Kondensation nicht mit Lysin-abgereichertem hG-CSF durchgeführt wird.

**9.** Verfahren nach Anspruch 8, umfassend die Kondensation von hG-CSF mit einer Carbonsäure der Formel (V)

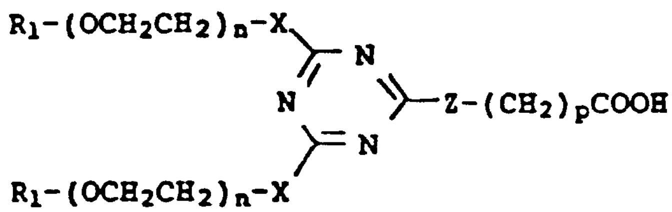

(V)

worin $R_1$, n und X die oben angegebenen Bedeutungen besitzen, Z für O, S oder NH steht und p für einen gegebenenfalls variablen, positiven, ganzzahligen Wert steht.

**10.** Verfahren nach Anspruch 8 oder 9, **dadurch gekennzeichnet, daß** man das Halogenid oder die aktive Carbonsäureverbindung zum Polypeptid in einem Anteil (Molverhältnis) entsprechend dem 2- bis 100-fachen der Menge an in dem Polypeptidmolekül vorliegenden Aminogruppen zugibt und das Gemisch bei einer Temperatur von 4 bis 37°C, vorzugsweise 4 bis 10°C, und bei einem pH von 7 bis 10 1 Stunde bis 2 Tage, vorzugsweise 1 bis 24 Stunden, reagieren läßt, wobei der gewünschte chemisch modifizierte hG-CSF produziert wird.

**11.** Verwendung von wenigstens einem modifizierten Polypeptid nach Anspruch 1 bis 5 zur Herstellung eines pharmazeutischen Mittels mit Leukozytenwachstum-fördernder Aktivität.

**Patentansprüche für folgenden Vertragsstaat : ES**

**1.** Verfahren zur Herstellung eines modifizierten Polypeptids mit Aktivität von humanem Granulozyten-Kolonie-stimulierenden Faktor (hG-CSF), umfassend ein Polypeptid mit hG-CSF-Aktivität, wobei wenigstens eine Aminosäuregruppe davon mit einer Gruppe der Formel

$$R_1\{OCR_2CH_2\}_nX\text{-}R_2 \qquad (I)$$

substituiert ist, worin
$R_1$ für eine Alkyl- oder Alkanoylgruppe steht;
n für einen gegebenenfalls variablen, positiven, ganzzahligen Wert steht;
X für O, NH oder S steht;
$R_2$ für

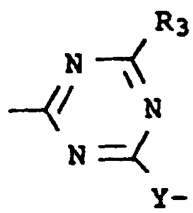

steht, [worin $R_3$ für OH, Cl, O-$(CH_2CH_2O)_n$-$R_1$ steht, worin $R_1$ und n die oben angegebenen Bedeutungen besitzen, Y fehlt oder für Z-$(CH_2)_p$CO steht, worin Z für O, S oder NH steht und p für einen gegebenenfalls variablen, positiven, ganzzahligen Wert steht]; oder für $(CO)_m$-$(CH_2)_1$CO steht, worin m für 0 oder 1 steht; und l für einen gegebenenfalls variablen, positiven, ganzzahligen Wert steht;
umfassend die Kondensation von hG-CSF mit einem Halogenid der Formel (II)

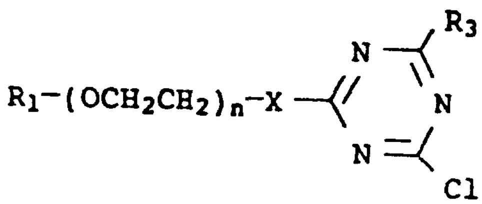

(II)

worin $R_1$, n, X und $R_3$ die oben angegebenen Bedeutungen besitzen; oder
die Kondensation von hG-CSF mit einer Carbonsäure der Formel (III)

$$R_1\{OCH_2CH_2)_n\text{-}X\text{-}(CO)_m(CH_2)_1\text{-}COOH \qquad (III)$$

worin $R_1$, n, X, m und l die oben angegebenen Bedeutungen besitzen; oder mit einer Carbonsäure der Formel (IV)

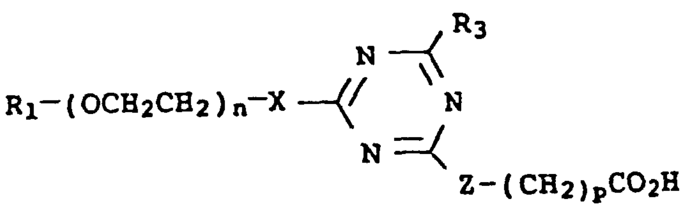

(IV)

worin $R_1$, n, Z, X, $R_3$ und p die oben angegebenen Bedeutungen besitzen, mit der Maßgabe, dass die Kondensation nicht mit Lysin-abgereichertem hG-CSF durchgeführt wird.

**2.** Verfahren nach Anspruch 1, unfassend die Kondensation von hG-CSF mit einer Carbonsäure der Formel (V)

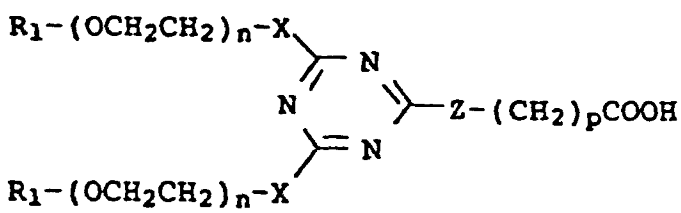

(V)

worin $R_1$, n und X die oben angegebenen Bedeutungen besitzen, Z für O, S oder NH steht und p für einen gegebenenfalls variablen, positiven, ganzzahligen Wert steht.

3. Verfahren nach Anspruch 1 oder 2, **dadurch gekennzeichnet, daß** man das Halogenid oder die aktive Carbonsäureverbindung zum Polypeptid in einem Anteil (Molverhältnis) entsprechend dem 2- bis 100-fachen der Menge an in dem Polypeptidmolekül vorliegenden Aminogruppen zugibt und das Gemisch bei einer Temperatur von 4 bis 37°C, vorzugsweise 4 bis 10°C, und bei einem pH von 7 bis 10 1 Stunde bis 2 Tage, vorzugsweise 1 bis 24 Stunden, reagieren läßt, wobei der gewünschte chemisch modifizierte hG-CSF produziert wird.

4. Verfahren gemäß einem der Ansprüche 1 bis 3, worin $R_1$ für eine $C_{1-18}$-Alkyl- oder -Alkanoylgruppe steht.

5. Verfahren gemäß einem der Ansprüche 1 bis 3, worin n für einen positiven, ganzzahligen Wert steht, der nicht größer als 500 ist.

6. Verfahren gemäß einem der Ansprüche 1 bis 3, worin l für einen positiven, ganzzahligen Wert steht, der nicht größer als 100 ist.

7. Verfahren gemäß einem der Ansprüche 1 bis 3, worin die Gruppe der Formel (I) ein Molekulargewicht von nicht mehr als 30000 besitzt.

8. Verfahren zur Herstellung eines pharmazeutisches Mittels, wobei man bei diesem Verfahren ein modifiziertes Polypeptid gemäß einem der Ansprüche 1 bis 7, gegebenenfalls in Kombination mit einem pharmazeutisch akzeptablen Träger bereitstellt.

9. Verfahren nach Anspruch 8, wobei man bei diesem Verfahren ein modifiziertes Polypeptid gemäß einem der Ansprüche 1 bis 7 in lyophilisierter Form bereitstellt.

**Patentansprüche für folgenden Vertragsstaat : GR**

1. Modifiziertes Polypeptid mit Aktivität von humanem Granulozyten-Kolonie-stimulierenden Faktor (hG-CSF), umfassend ein Polypeptid mit hG-CSF-Aktivität, wobei wenigstens eine Aminosäuregruppe davon mit einer Gruppe der Formel

$$R_1\{OCH_2CH_2\}_nX\text{-}R_2\text{-} \qquad (I)$$

substituiert ist, worin
$R_1$ für eine Alkyl- oder Alkanoylgruppe steht;
n für einen gegebenenfalls variablen, positiven, ganzzahligen Wert steht;
X für O, NH oder S steht;
$R_2$ für

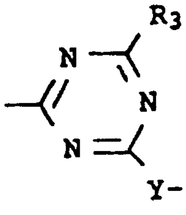

steht, [worin $R_3$ für OH, Cl, O-$(CH_2CH_2O)_n$-$R_1$ steht, worin $R_1$ und n die oben angegebenen Bedeutungen besitzen, Y fehlt oder für Z-$(CH_2)_p$CO steht, worin Z für O, S oder NH steht und p für einen gegebenenfalls variablen, positiven, ganzzahligen Wert steht]; oder für $(CO)_m$-$(CH_2)_1$CO steht, worin m für 0 oder 1 steht; und l für einen gegebenenfalls variablen, positiven, ganzzahligen Wert steht, mit der Maßgabe, dass das modifizierte Polypeptid nicht aus Lysin-abgereichertem hG-CSF hergestellt ist.

2. Modifiziertes Polypeptid nach Anspruch 1, worin $R_1$ für eine $C_{1-18}$-Alkyl- oder -Alkanoylgruppe steht.

**3.** Modifiziertes Polypeptid nach Anspruch 1, worin n für einen positiven, ganzzahligen Wert steht, der nicht größer als 500 ist.

**4.** Modifiziertes Polypeptid nach Anspruch 1, worin l für einen positiven, ganzzahligen Wert steht, der nicht größer als 100 ist.

**5.** Modifiziertes Polypeptid nach Anspruch 1, worin die Gruppe der Formel (I) ein Molekulargewicht von nicht mehr als 30000 besitzt.

**6.** Verfahren zur Herstellung eines pharmazeutisches Mittels, wobei man bei diesem Verfahren ein modifiziertes Polypeptid gemäß einem der Ansprüche 1 bis 5, gegebenenfalls in Kombination mit einem pharmazeutisch akzeptablen Träger bereitstellt.

**7.** Verfahren nach Anspruch 6, wobei man bei diesem Verfahren ein modifiziertes Polypeptid gemäß einem der Ansprüche 1 bis 5 in lyophilisierter Form bereitstellt.

**8.** Verfahren zur Herstellung eines chemisch modifizierten hG-CSF gemäß einem der Ansprüche 1 bis 5, umfassend die Kondensation von hG-CSF mit einem Halogenid der Formel (II)

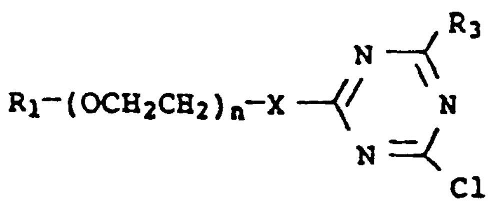

(II)

worin $R_1$, n, X und $R_3$ die oben angegebenen Bedeutungen besitzen; oder
die Kondensation von hG-CSF mit einer Carbonsäure der Formel (III)

$$R_1\{OCH_2CH_2)_n\text{-}X\text{-}(CO)_m(CH_2)_1\text{-}COOH \qquad (III)$$

worin $R_1$, n, X, m und l die oben angegebenen Bedeutungen besitzen; oder mit einer Carbonsäure der Formel (IV)

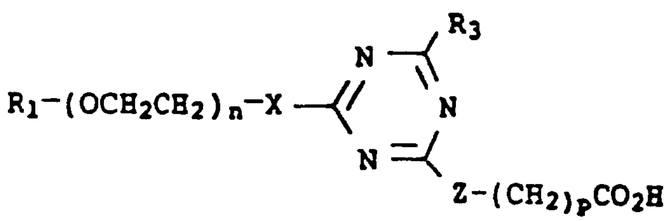

(IV)

worin $R_1$, n, Z, X, $R_3$ und p die oben angegebenen Bedeutungen besitzen, mit der Maßgabe, dass die Kondensation nicht mit Lysin-abgereichertem hG-CSF durchgeführt wird.

**9.** Verfahren nach Anspruch 8, unfassend die Kondensation von hG-CSF mit einer Carbonsäure der Formel (V)

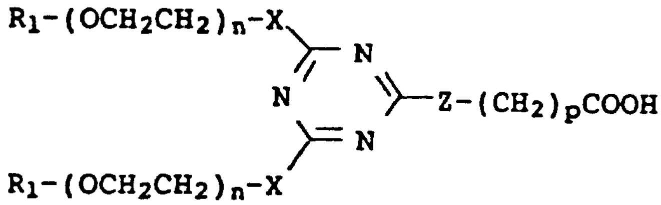

(V)

worin $R_1$, n und X die oben angegebenen Bedeutungen besitzen, Z für O, S oder NH steht und p für einen gegebenenfalls variablen, positiven, ganzzahligen Wert steht.

10. Verfahren nach Anspruch 8 oder 9, **dadurch gekennzeichnet, daß** man das Halogenid oder die aktive Carbonsäureverbindung zum Polypeptid in einem Anteil (Molverhältnis) entsprechend dem 2- bis 100-fachen der Menge an in dem Polypeptidmolekül vorliegenden Aminogruppen zugibt und das Gemisch bei einer Temperatur von 4 bis 37°C, vorzugsweise 4 bis 10°C, und bei einem pH von 7 bis 10 1 Stunde bis 2 Tage, vorzugsweise 1 bis 24 Stunden, reagieren läßt, wobei der gewünschte chemisch modifizierte hG-CSF produziert wird.

11. Verwendung von wenigstens einem modifizierten Polypeptid nach Anspruch 1 bis 5 zur Herstellung eines pharmazeutischen Mittels mit Leukozytenwachstum-fördernder Aktivität.

**Revendications**

**Revendications pour les Etats contractants suivants : AT, BE, CH, DE, FR, GB, IT, LI, LU, NL, SE**

1. Polypeptide modifié ayant une activité de facteur de stimulation des colonies de granulocytes humain (hG-CSF), comprenant un polypeptide ayant une activité de hG-CSF, dont au moins un groupe amino est substitué par un groupe de formule

$$R_1\text{-}(OCH_2CH_2)_n\text{-}X\text{-}R_2 \qquad (I)$$

dans laquelle $R_1$ est un groupe alkyle ou alcanoyle; n est un entier positif éventuellement variable; X est O, NH ou S; $R_2$ est

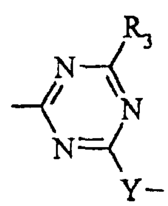

[où $R_3$ est OH, Cl, O-$(CH_2CH_2O)_n$-$R_1$, $R_1$ et n ayant la définition donnée ci-dessus, Y peut ne pas être présent ou représente Z-$(CH_2)_p$CO, où Z est O, S ou NH et p est un entier positif éventuellement variable], ou $(CO)_m$-$(CH_2)_1$CO, où m est 0 ou 1; l est entier positif éventuellement variable; pour autant que ledit polypeptide modifié n'est pas préparé à partir de hG-CSF dépourvu de lysine.

2. Polypeptide modifié selon la revendication 1, dans lequel $R_1$ est un groupe alkyle ou alcanoyle en $C_1$-$C_{18}$.

3. Polypeptide modifié selon la revendication 1, dans lequel n est un entier positif inférieur ou égal à 500.

4. Polypeptide modifié selon la revendication 1, dans lequel l est un entier positif inférieur ou égal à 100.

5. Polypeptide modifié selon la revendication 1, dans lequel le groupe de formule (I) a une masse moléculaire inférieure ou égale à 30 000.

**6.** Composition pharmaceutique comprenant un polypeptide modifié selon l'une quelconque des revendications 1 à 5, éventuellement en combinaison avec un support pharmaceutiquement acceptable.

**7.** Composition pharmaceutique selon la revendication 6, contenant le polypeptide modifié selon l'une quelconque des revendications 1 à 5 sous forme lyophilisée.

**8.** Procédé de préparation d'un hG-CSF chimiquement modifié selon l'une quelconque des revendications 1 à 5, comprenant la condensation de hG-CSF avec un halogénure de formule (II)

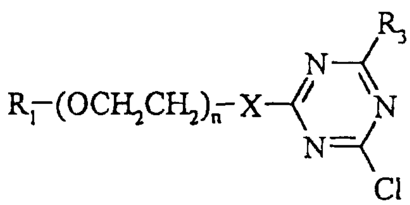

(II)

dans laquelle $R_1$, n, X et $R_3$ sont tels que définis précédemment; ou la condensation de hG-CSF avec un acide carboxylique de formule (III)

$$R_1\text{-}(OCH_2CH_2)_n\text{-}X\text{-}(CO)_m(CH_2)_1\text{-}COOH \qquad (III)$$

dans laquelle $R_1$, n, X, m et l son tels que définis précédemment; ou avec un acide carboxylique de formule (IV)

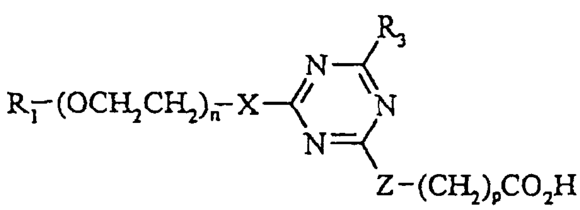

(IV)

dans laquelle $R_1$, n, Z, X, $R_3$ et p sont tels que définis précédemment ; pour autant que la condensation n'est pas réalisée avec hG-CSF dépourvu de lysine.

**9.** Procédé selon la revendication 8, comprenant la condensation de hG-CSF avec un acide carboxylique représenté par la formule (V)

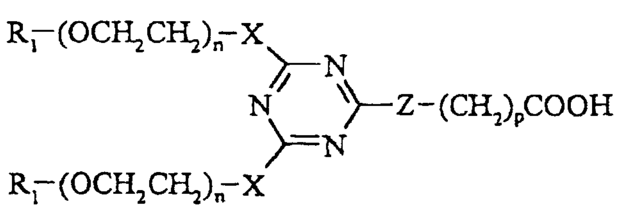

(V)

dans laquelle $R_1$, n et X sont tels que définis ci-dessus, Z est O, S ou NH et p est un entier positif éventuellement variable.

**10.** Procédé selon la revendication 8 ou 9, **caractérisé en ce que** l'on ajoute l'halogénure ou l'acide carboxylique actif au polypeptide en une proportion (rapport molaire) de 2 à 100 fois la quantité de groupes amino présents dans la molécule du polypeptide, et **en ce qu'**on laisse le mélange réagir à une température de 4 à 37°C, de préférence

de 4 à 10°C, et à un pH de 7 à 10 pendant 1 heure à 2 jours, de préférence pendant 1 à 24 heures, grâce à quoi on produit le hG-CSF chimiquement modifié désiré.

**11.** Utilisation d'au moins un polypeptide modifié selon les revendications 1 à 5 pour la préparation d'une composition pharmaceutique manifestant une activité de promotion de la croissance des leucocytes.

**Revendications pour l'Etat contractant suivant : ES**

**1.** Procédé de préparation d'un polypeptide modifié ayant une activité de facteur de stimulation des colonies de granulocytes humain (hG-CSF), comprenant un polypeptide ayant une activité de hG-CSF, dont au moins un groupe amino est substitué par un groupe de formule

$$R_1\text{-}(OCH_2CH_2)_n\text{-}X\text{-}R_2 \qquad \text{(I)}$$

dans laquelle $R_1$ est un groupe alkyle ou alcanoyle; n est un entier positif éventuellement variable; X est O, NH ou S; $R_2$ est

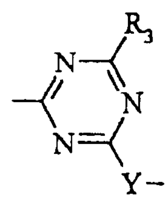

[où $R_3$ est OH, Cl, O-$(CH_2CH_2O)_n$-$R_1$, $R_1$ et n ayant la définition donnée ci-dessus, Y peut ne pas être présent ou représente Z-$(CH_2)_p$CO, où Z est O, S ou NH et p est un entier positif éventuellement variable], ou $(CO)_m$-$(CH_2)_1$CO, où m est 0 ou 1; l est un entier positif éventuellement variable;

le procédé comprenant la condensation de hG-CSF avec un halogénure de formule (II)

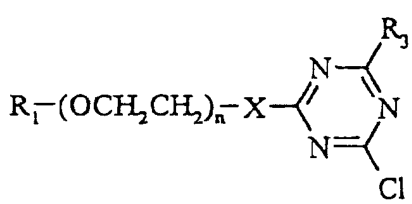

**(II)**

dans laquelle $R_1$, n, X et $R_3$ sont tels que définis précédemment; ou la condensation de hG-CSF avec un acide carboxylique de formule (III)

$$R_1\text{-}(OCH_2CH_2)_n\text{-}X\text{-}(CO)_m(CH_2)_1\text{-}COO \qquad \text{(III)}$$

dans laquelle $R_1$, n, X, m et l sont tels que définis précédemment; ou avec un acide carboxylique de formule (IV)

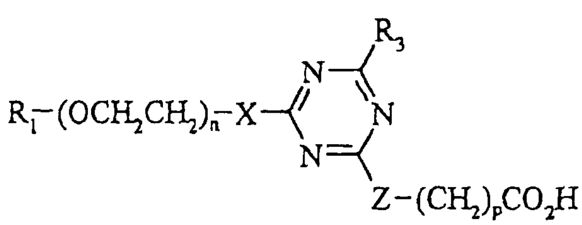

**(IV)**

dans laquelle $R_1$, n, Z, X, $R_3$ et p sont tels que définis précédemment ; pour autant que la condensation n'est pas réalisée avec hG-CSF dépourvu de lysine.

**2.** Procédé selon la revendication 1, comprenant la condensation de hG-CSF avec un acide carboxylique représenté par la formule (V)

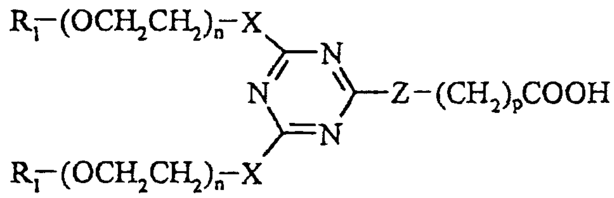

(V)

dans laquelle $R_1$, n et X sont tels que définis ci-dessus, Z est O, S ou NH et p est un entier positif éventuellement variable.

**3.** Procédé selon la revendication 1 ou 2, **caractérisé en ce que** l'on ajoute l'halogénure ou l'acide carboxylique actif au polypeptide en une proportion (rapport molaire) de 2 à 100 fois la quantité de groupes amino présents dans la molécule du polypeptide, et **en ce qu'**on laisse le mélange réagir à une température de 4 à 37°C, de préférence de 4 à 10°C, et à un pH de 7 à 10 pendant 1 heure à 2 jours, de préférence pendant 1 à 24 heures, grâce à quoi on produit le hG-CSF chimiquement modifié désiré.

**4.** Procédé selon l'une des revendications 1 à 3, dans lequel $R_1$ est un groupe alkyle ou alcanoyle en $C_1$-$C_{18}$.

**5.** Procédé selon l'une des revendications 1 à 3, dans lequel n est un entier positif inférieur ou égal à 500.

**6.** Procédé selon l'une des revendications 1 à 3, dans lequel l est un entier positif inférieur ou égal à 100.

**7.** Procédé selon l'une des revendications 1 à 3, dans lequel le groupe de formule (I) a une masse moléculaire inférieure ou égale à 30 000.

**8.** Procédé de préparation d'une composition pharmaceutique, ce procédé comprenant la fourniture d'un polypeptide modifié tel que défini dans l'une quelconque des revendications 1 à 7, éventuellement en combinaison avec un support pharmaceutiquement acceptable.

**9.** Procédé selon la revendication 8, ce procédé comprenant la fourniture du polypeptide modifié tel que défini dans l'une quelconque des revendications 1 à 7 sous forme lyophilisée.

**Revendications pour l'Etat contractant suivant : GR**

**1.** Polypeptide modifié ayant une activité de facteur de stimulation des colonies de granulocytes humain (hG-CSF), comprenant un polypeptide ayant une activité de hG-CSF, dont au moins un groupe amino est substitué par un groupe de formule

$$R_1\text{-}(OCH_2CH_2)_n\text{-}X\text{-}R_2 \qquad (I)$$

dans laquelle $R_1$ est un groupe alkyle ou alcanoyle; n est un entier positif éventuellement variable; X est O, NH ou S; $R_2$ est

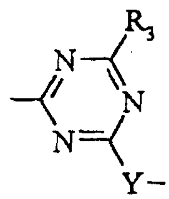

[où $R_3$ est OH, Cl, O-$(CH_2CH_2O)_n$-$R_1$, $R_1$ et n ayant la définition donnée ci-dessus, Y peut ne pas être présent ou représente Z-$(CH_2)_p$CO où Z est O, S ou NH et p est un entier positif éventuellement variable], ou $(CO)_m$-$(CH_2)_1$CO, où m est 0 ou 1; l est un entier positif éventuellement variable; pour autant que ledit polypeptide modifié n'est pas préparé à partir de hG-CSF déporvu de lysine.

2. Polypeptide modifié selon la revendication 1, dans lequel $R_1$ est un groupe alkyle ou alcanoyle en $C_1$-$C_{18}$.

3. Polypeptide modifié selon la revendication 1, dans lequel n est un entier positif inférieur ou égal à 500.

4. Polypeptide modifié selon la revendication 1, dans lequel l est un entier positif inférieur ou égal à 100.

5. Polypeptide modifié selon la revendication 1, dans lequel le groupe de formule (I) a une masse moléculaire inférieure ou égale à 30 000.

6. Procédé de préparation d'une composition pharmaceutique, le procédé comprenant la fourniture d'un polypeptide modifié tel que défini dans l'une quelconque des revendications 1 à 5, éventuellement en combinaison avec un support pharmaceutiquement acceptable.

7. Procédé selon la revendication 6, ce procédé comprenant la fourniture du polypeptide modifié tel que défini dans l'une quelconque des revendications 1 à 5 sous forme lyophilisée.

8. Procédé de préparation d'un hG-CSF chimiquement modifié selon l'une quelconque des revendications 1 à 5, comprenant la condensation de hG-CSF avec un halogénure de formule (II)

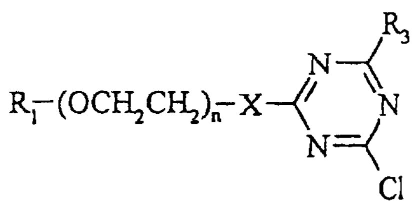

(II)

dans laquelle $R_1$, n, X et $R_3$ sont tels que définis précédemment; ou la condensation de hG-CSF avec un acide carboxylique de formule (III)

$$R_1\text{-}(OCH_2CH_2)_n\text{-}X\text{-}(CO)_m\text{-}COOH \qquad \text{(III)}$$

dans laquelle $R_1$, n, X, m et l sont tels que définis précédemment; ou avec un acide carboxylique de formule (IV)

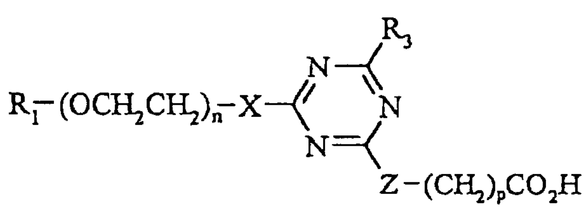

(IV)

dans laquelle $R_1$, n, Z, X, $R_3$ et p sont tels que définis précédemment ; pour autant que la condensation n'est pas réalisée avec hG-CSF dépourvu de lysine.

**9.** Procédé selon la revendication 8, comprenant la condensation de hG-CSF avec un acide carboxylique représenté par la formule (V)

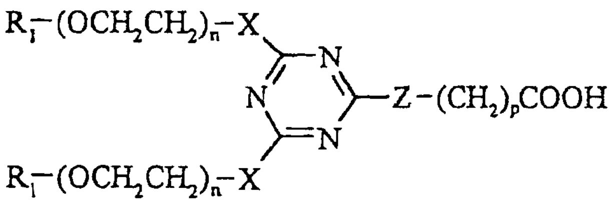

(V)

dans laquelle $R_1$, n et X sont tels que définis ci-dessus, Z est O, S ou NH et p est un entier positif éventuellement variable.

**10.** Procédé selon la revendication 8 ou 9, **caractérisé en ce que** l'on ajoute l'halogénure ou l'acide carboxylique actif au polypeptide en une proportion (rapport molaire) de 2 à 100 fois la quantité de groupes amino présents dans la molécule du polypeptide, et **en ce qu'**on laisse le mélange réagir à une température de 4 à 37°C, de préférence de 4 à 10°C et à un pH de 7 à 10 pendant 1 heure à 2 jours, de préférence pendant 1 à 24 heures, grâce à quoi on produit le hG-CSF chimiquement modifié désiré.

**11.** Utilisation d'au moins un polypeptide modifié selon les revendications 1 à 5 pour la préparation d'une composition pharmaceutique manifestant une activité de promotion de la croissance des leucocytes.